# EUROPÄISCHE PATENTSCHRIFT

(11) **EP 3 426 764 B1**
(45) Veröffentlichungstag und Bekanntmachung des Hinweises auf die Patenterteilung: **27.08.2025**
(21) Anmeldenummer: 17712929.3
(22) Anmeldetag: 07.03.2017
(51) Int. Cl.: C12P 23/00, C12M 1/00, C10L 3/08, C10L 3/10, C12P 5/00

(54) **REGENERATIVE SYNTHESE VON CHEMISCHEN ENERGIESPEICHERN UND FEINCHEMIKALIEN**
REGENERATIVE SYNTHESIS OF CHEMICAL STORED-ENERGY SOURCES AND FINE CHEMICALS
SYNTHÈSE RENOUVELABLE DE RÉSERVOIRS D'ÉNERGIE CHIMIQUES ET DE PRODUITS DE CHIMIE FINE

(30) Priorität: 09.03.2016 DE 102016203889
(43) Veröffentlichungstag der Anmeldung: 16.01.2019
(73) Patentinhaber: Fraunhofer-Gesellschaft zur Förderung der angewandten Forschung e.V., 80686 München (DE)
(72) Erfinder: CSEPEI, Lenard-Istvan, 94315 Straubing (DE); STEFFLER, Fabian, 04155 Leipzig (DE); GÄRTNER, Tobias, 93093 Donaustauf (DE); SIEBER, Volker, 85405 Nandlstadt (DE)
(74) Vertreter: Schrell, Andreas
(86) Internationale Anmeldenummer: PCT/EP2017/055303
(87) Internationale Veröffentlichungsnummer: WO 2017/153396

(56) Entgegenhaltungen:
- EP-A1- 1 454 991
- EP-A1- 2 940 123
- WO-A2-2008/039499
- DE-A1- 102004 006 825
- DE-A1- 102011 112 093
- US-A1- 2015 225 743
- FRANK SONNTAG ET AL: "Engineering Methylobacterium extorquens for de novo synthesis of the sesquiterpenoid [alpha]-humulene from methanol", METABOLIC ENGINEERING, vol. 32, 30 November 2015 (2015-11-30), US, pages 82 - 94, XP055271914, ISSN: 1096-7176, DOI: 10.1016/j.ymben.2015.09.004
- ORITA IZUMI ET AL: "Biosynthesis of polyhydroxyalkanoate copolymers from methanol byMethylobacterium extorquensAM1 and the engineered strains under cobalt-deficient conditions", APPLIED MICROBIOLOGY AND BIOTECHNOLOGY, SPRINGER, DE, vol. 98, no. 8, 30 January 2014 (2014-01-30), pages 3715 - 3725, XP035328998, ISSN: 0175-7598, [retrieved on 20140116], DOI: 10.1007/S00253-013-5490-9
- S. J. VAN DIEN ET AL: "Genetic Characterization of the Carotenoid Biosynthetic Pathway in Methylobacterium extorquens AM1 and Isolation of a Colorless Mutant", APPLIED AND ENVIRONMENTAL MICROBIOLOGY, vol. 69, no. 12, 31 December 2003 (2003-12-31), pages 7563 - 7566, XP055273125, ISSN: 0099-2240, DOI: 10.1128/AEM.69.12.7563-7566.2003

## Beschreibung

Die Erfindung betrifft Vorrichtungen und Verfahren zur Herstellung von chemischen Energiespeichern in Form energiereicher organischer Verbindungen sowie von Feinchemikalien aus regenerativen und biogenen Quellen, besonders aus Kohlenstoffdioxid-haltigen Substraten, besonders zur Anwendung in dezentralen Anlagen, zum Beispiel Anlagen, die mit Strom aus regenerativen Quellen betrieben werden.

Die Umwandlung und Speicherung von Energie aus regenerativen Quellen, besonders von Kohlenstoffdioxid (CO2), in energiereicher organischen Molekülen (CO2-Fixierung) stellt eine große Herausforderung dar. Es sollen organische Restströme und insbesondere das Abgas CO2 stofflich genutzt und daraus Chemikalien, besonders neue chemische Energiespeicher oder Produkte mit insgesamt positiver Energiebilanz synthetisiert werden. Die Umwandlung in kurz- oder längerkettige Kohlenwasserstoffe als Energieträger steht dabei bisher im Vordergrund. Alternativ oder zusätzlich könnten die Substrate aus regenerativen Quellen auch direkt in verwertbare Feinchemikalien umgewandelt werden. Feinchemikalien können für die weitere Synthese komplexer Verbindungen, beispielsweise von Biopolymeren zur Verfügung stehen.

Regenerative und vor allem biogene Quellen sind aber systembedingt häufig dezentral anzutreffen, beispielsweise in Form von Abgasreinigern einzelner Industrieanlagen und Kraftwerken oder als Biogasanlagen. Es ist daher wünschenswert, die Umwandlung und Speicherung der Energie daraus ebenfalls dezentral und in lokalen Kleinanlagen durchführen zu können. Bekannte Verfahren, wie die Fischer-Tropsch-Synthese zur Neu-Herstellung organischer Verbindungen aus Synthesegasen sind demgegenüber aufwändig und vor allem für dezentrale Anlagen, insbesondere für die Verwertung relativ kleiner Gasmengen aus Biogasanlagen oder aus der Abgasreinigung nicht einsetzbar. Daneben besitzen bekannte Anlagen und Verfahren eine schlechte Gesamtenergiebilanz, da sie insbesondere eine rein auf die Endproduktausbeute und -qualität ausgerichtete

Verfahrensführung einsetzen.

DE 10 2011 112093 offenbart einen Niederdruck-Methanolreaktor zur Erzeugung von Methanol aus Synthesegas über eine Kupfer-katalysierte heterogene Katalyse bei Temperaturen von 200-300 °C. Dem Reaktor sind des Weiteren ein Methanolspeicher und Wasserabscheider, Wäscher sowie Kondensationseinrichtungen zugeordnet.

FRANK SONNTAG ET AL: "Engineering Methylobacterium extorquens for de novo synthesis of the sesquiterpenoid [alpha]-humulene from methanol", METABOLIC ENGINEERING, Bd. 32, 1. November 2015, offenbart eine Vorrichtung und ein Verfahren zur aeroben Fermentation von Methanol an Methanol-fixierenden Methylobacter Mikroorganismen zur Herstellung von Terpenen als chemischer Energiespeicher.

Der vorliegenden Erfindung liegt das technische Problem zugrunde, neue Verfahren und Vorrichtungen für die Synthese von Feinchemikalien und chemischen Energiespeichern aus regenerativen, insbesondere CO2-haltigen Substraten bereitzustellen, die auch in integralen Anlagen und Kleinmodulen und hocheffizient betrieben werden können und eine günstige Gesamtenergiebilanz aufweisen, weil der Energieeintrag in das System gering ist.

Die Erfindung stellt dazu Verfahren und Mittel in Form einer gut skalierbare Komplettanlage zur direkten integralen, das heißt kombinierten chemokatalytischen und biotechnologischen Synthese von chemischen Energiespeichern aus CO2-haltigen Synthesegasgemischen bereit. Die Erfindung macht sich die Erkenntnis zu Nutze, dass auf chemokatalytischen Wege aus CO2- und H2-haltigen Gasgemischen, wie sie beispielsweise in Biogasanlagen oder Abgasreinigung und durch Elektrolyse regenerativ erhalten werden können, zunächst bei Atmosphärendruck oder Niederdruck primär Methanol-haltige Substrate synthetisiert werden können, die dann unmittelbar, vor allem ohne weitere Aufreinigungs- oder Zwischenlagerschritte, als Substrat für primär Methanol-fixierende Mikroorganismen in einer aeroben Fermentation dienen.
Durch diesen der chemischen Synthese des Methanol-haltigen Substrats unmittelbar nachschaltbaren biotechnologischen Prozess können Produkte und Produktgemische erhalten werden, die in Form energiereicher organischer Moleküle als chemische Energiespeicher und/oder unmittelbar als Feinchemikalien dienen können. Der integrale Aufbau der Gesamtanlage erlaubt vorteilhafterweise zusätzlich, entstehende Reaktionswärme in dem System selbst effizient wiederzuverwerten, insbesondere zur Vorheizung des Synthesegases und/oder zur Temperierung des Zellkulturmediums in dem biotechnologischen Prozess. Auf Zufuhr externer Energie, beispielsweise elektrischer Energie, zur dauerhaften Heizung und/oder Kühlung einzelner Komponenten der Anlage kann daher verzichtet werden.

Erster Aspekt der Erfindung ist daher eine Vorrichtung, das heißt eine Gesamtanlage, zur integralen Synthese von organischen Energiespeichermolekülen oder Feinchemikalien aus einem CO2- und H2-haltigen Synthesegasgemischen, wobei die Vorrichtung erfindungsgemäß zumindest folgende, in Stoffflussrichtung unmittelbar hintereinander geschaltete Elemente aufweist: (1) Niederdruckgasreaktor, das heißt besonders einen Methanolsynthesereaktor, welcher einen insbesondere kupferbasierten heterogenen Katalysator enthält, zum Zwecke der katalytischen Synthese eines Methanol-haltigen Produktgemisches aus dem CO2- und H2-haltigen Synthesegas; (2) Kondensator oder Kühlfalle, dem Niederdruckgasreaktor, insbesondere unmittelbar nachgeschaltet, zur Verflüssigung des in dem Niederdruckgasreaktor erhaltenen Methanol-haltigen Gases zu einem flüssigen Methanol-haltigen Substrat, wobei insbesondere die nicht verflüssigbaren, das heißt nicht kondensierbaren Restgase aus dem Methanol-haltigen Gas abtrennbar und insbesondere in den Niederdruckgasreaktor rückführbar sind; (3) Fermentationsreaktor, dem Kondensator, unmittelbar nachgeschaltet, der flüssiges Zellkulturmedium und spezielle Methanol-fixierende Mikroorganismen enthält zum Zwecke der aeroben Fermentation der Methanol-haltigen Substrate zu den organischen Energiespeichern oder Feinchemikalien.

Bevorzugt enthält diese Vorrichtung auch eine dem Niederdruckgasreaktor oder Methanolsynthesereaktor, insbesondere unmittelbar vorgeschaltete Gasdosiereinheit zum Zwecke der Dosierung der CO2-und H2-haltigen Synthesegase in den Niederdruckgasreaktor. Die Gasdosiereinheit steuert die Stofflüsse in die Synthesereaktion und damit bevorzugt den Gesamtprozess. Bevorzugt ist dieser Gasdosiereinheit zusätzlich eine Gasspeichereinheit zum Zwecke der Zwischenspeicherung der CO2-und H2-haltigen Synthesegase, insbesondere unmittelbar vorgeschaltet. Zum Zwecke der Rückführung von nicht verflüssigbaren Restgasen, besonders CO2, CO2-haltiges und DME, aus dem Kondensator ist dabei bevorzugt eine Rückleitung zwischen Kondensator und dieser Gasspeichereinheit vorgesehen. So kann vorteilhafterweise gewährleistet werden, dass die Restgase erneut durch den Niederdruckgasreaktor geführt werden, um den Umsatz der katalytischen Synthese zu Methanol-haltigen Gasen und letztlich zu dem Methanol-haltigen Substrat zu erhöhen.

Die erfindungsgemäße integrale unmittelbare Verschaltung dieser Komponenten erlaubt, dass die bei der katalytischen Synthese des Methanol-haltigen Gases in dem Niederdruckgasreaktor entstehende Reaktionswärme unmittelbar in der Vorrichtung selbst genutzt werden kann. Dies geschieht zum einen durch einen dem Niederdruckgasreaktor vorgeschalteten oder in diesen integrierten Gasheizer, welcher erfindungsgemäß bevorzugt über einen mit dem Niederdruckgasreaktor verbundenen Wärmetauscher, welche Reaktionswärme aus dem Niederdruckgasreaktor abführt, gespeist wird, um das Synthesegas vor Eintritt in den Reaktor auf eine günstige Reaktionstemperatur zu bringen. Zum anderen ist bevorzugt oder alternativ vorgesehen, dass der Fermentationsreaktor, welcher das Zellkulturmedium enthält, über einen diesem zugeordneten oder integrierten Medienheizer temperiert wird, um die Kultivierungsbedingungen für die dort enthaltenen Methanol-fixierenden Mikroorganismen konstant im Optimum zu halten. Dabei wird der Medienheizer über die Abwärme aus dem Kondensator, besonders über einen an dem Kondensator angeordneten Kühlmantel und Wärmetauscher, gespeist.

Die Erfindung betrifft auch einen speziell konstruierten neuartigen Kondensator zur effizienten Kondensation, das heißt Verflüssigung, von Methanol-haltigen Gasen, der einen bevorzugten Bestandteil der erfindungsgemäßen Vorrichtung bilden kann. Dieser besteht aus einer insbesondere zylindrischen länglichen Kammer, die im Betrieb primär lotrecht orientiert ist. In der Kammer existiert ein oberer Gas-Abschnitt ausgebildet, der im Betrieb gasgefüllt ist, und ein unterer Flüssigkeits-Abschnitt, der im Betrieb flüssigkeitsgefüllt ist. An der Kammer, im Bereich des oberen Gas-Abschnitts befindet sich zumindest ein oberer Anschluss zum Ableiten nicht verflüssigbarer Restgase. In dem unteren Flüssigkeits-Abschnitt ist ein unterer Anschluss ausgebildet, zum Abführen des in dem Kondensator gebildeten Methanol-haltigen Substrates. Über einen weiteren Anschluss können gasförmige Methanol-haltige Gemische, wie sie insbesondere aus dem unmittelbar vorgeschalteten Niederdruckgasreaktor stammen, in die Kammer geleitet werden, wobei dieser Anschluss in ein Gasrohr im Inneren der Kammer mündet, welches erfindungsgemäß in den Flüssigkeits-Abschnitt der Kammer und damit im Betrieb in die dort vorhandene Flüssigkeit eintaucht. Bevorzugt ist dabei vorgesehen, dass das Gasrohr aus wärmeleitendem Material, Kunststoff oder Metall ausgebildet ist, und zusätzlich schraubenartig oder spiralig gebogen ist, um den Kontakt des Gasrohrs mit der im vorhandenen Flüssigkeit zu vergrößern. Die Kammer ist mit einem Kühlmantel umgeben. Die im Betrieb in der Kammer vorgelegte Flüssigkeit, insbesondere, Wasser wird gekühlt, die durch Einleiten von heißem Gas in die Flüssigkeit eingebrachte Wärme abgeführt. Ohne an die Theorie gebunden sein zu wollen, kühlt das Methanol-haltige Gas beim Durchlaufen des eintauchenden Gasrohrs aufgrund der dieses umgebenden kühleren Flüssigkeit ab und verlässt teilweise bereits flüssig das untere offene Ende des Gasrohrs. Auf diese Weise wird vorteilhafterweise das Methanol-haltige Gas nach der Art einer Kühlfalle in der dort vorhandenen Flüssigkeit insbesondere Wasser, kondensiert. Es wird so eine wässrige Lösung des Methanol-haltigen Substrats erhalten. Nicht kondensierbares Restgas steigt aus dieser Flüssigkeit nach oben und kann durch den weiteren oberen Anschluss zum Ableiten der nicht verflüssigbaren Restgase aus der Kammer abgezogen oder durch inertes Spülgas (N2) ausgetrieben werden.

Der Fermentationsreaktor der Vorrichtung ist in an sich bekannter Weise aufgebaut. Bevorzugt sind die Methanol-fixierenden Mikroorganismen ausgewählt aus Bakterien der Gattungen Methylobacterium, Cupriavidus, Methylocella, oder ausgewählt aus Hefen der Gattung Pichia. Daneben sind weitere Mikroorganismen einsetzbar, welche in der Lage sind, Methanol als insbesondere einzige Kohlenstoffquelle zu verwerten und daraus organische Moleküle, welches als chemische Energiespeicher und/oder Feinchemikalien dienen können, aufzubauen. Bevorzugt aber sind die Mikroorganismen ausgewählt aus Methylobacterium sp. , zum Beispiel Methylobacterium extorquens (AM1). Auch solche Mikroorganismen sind einsetzbar, die nicht ursprünglich Methanol verwerten, sondern noch dahingehend gezüchtet werden.

In einer bevorzugten Ausführung ist dem Fermentationsreaktor noch eine Ernte-/ und Stofftrenneinheit nachgeschaltet, oder alternativ in diesen integriert. Diese ist spezifisch ausgebildet, die dort biotechnologisch synthetisierten organischen Energiespeichermoleküle oder Feinchemikalien, besonders Terpene oder Terpen-haltige Produktgemische, aus dem Zellkulturmedium oder den Zellen abzutrennen, wobei die Produkte dann isoliert erhalten werden können. Im Falle nicht löslicher oder nicht unmittelbar in das Zellkulturmedium abgegebener Produkte, sieht die Stofftrenneinheit eine Abtrennung der Zellen aus dem Zellkulturmedium (Ernte) und den Aufschluss der Zellen zur Freigabe der biotechnologisch gebildeten Produkte in an sich bekannter Weise vor. Weitere Produkte sind Bio-Kunststoffe, Bio-Additive und -Vorstufen davon (PHB) sowie Nahrungs- und Futtermittel oder Zusatzstoffe wie Proteine, Öle und Fette.

In praktischer Ausgestaltung ist vorgesehen, die Vorrichtung dazu spezifisch ausgebildet und deren Steuerung entsprechend programmiert, dass der Niederdruckreaktor primär kontinuierlich betrieben wird, die Verflüssigung des Methanol-haltigen Substrats, unter optionaler Rückführung der Restgase in den katalytischen Prozess, batch-weise betrieben wird und die Fermentation im fed-batch Betrieb läuft, wobei aus dem Kondensator periodisch Methanol-haltiges Substrat in den Fermentationsreaktor dosiert wird. Nach Abschluss der Fermentation wird das Endprodukt oder -Produktgemisch aus den Mikroorganismen und/oder dem Kulturmedium abgetrennt und der Fermentationsreaktor neu beschickt und beimpft. Dazu sind weist die Vorrichtung zusätzliche an sich bekannte Ventile und Fluidverbindungen in spezifischer Verschaltung auf. In einer besonderen Variante sind an dem Kondensator eine Ventilanordnung und/oder zusätzliche Anschlüsse vorgesehen, um in einem vorbereitenden Schritt Flüssigkeit, insbesondere Wasser in dem Flüssigkeits-Abschnitt der Kammer vorzulegen. Die Kondensation des Methanol-haltigen Substrats erfolgt in dieser Flüssigkeit. Die letztlich mit Methanol-haltigem Substrat angereicherte vorgelegte Flüssigkeit wird periodisch entleert. Über die Ventilanordnung und/oder zusätzliche Anschlüsse können das Innere der Kammer mit Inertgas gespült und die Restgase ausgetrieben werden. In einer Variante sind zwischen Kondensator und Fermentationsreaktor eine Ventilanordnung und/oder Dosiereinrichtung vorgesehen, die ein kontrolliertes Dosieren des Methanol-haltigem Substrats in die Fermentation erlauben.

Unter einem "CO2-haltigen Synthesegas" wird hier ein Gemisch mit den Hauptbestandteilen Wasserstoff (H2) und Kohlenstoffdioxid (CO2) verstanden. Diese liegen darin vorzugsweise in einem molaren Verhältnis von 3 Teilen H2 zu 1 Teil CO2 vor. Weitere Bestandteile dieses Synthesegases können unter anderem Kohlenstoffmonoxidgas (CO) aber auch Methan (CH4) und andere flüchtige organische Substanzen sein, wie sie beispielsweise in Biogasanlagen vorkommen. Die Gegenwart von Sauerstoff (O2) ist vorzugsweise zu vermeiden. Vorteilhafterweise kann solches CO2-haltiges Synthesegas unmittelbar aus dem Gasgemisch einer Biogasanlage gewonnen werden. CO2 kann alternativ oder zusätzlich als Produkt alkoholischer Gärung gewonnen werden. Alternativ oder zusätzlich können Anteile von Methan (CH4) in einem Biogas, besonders durch Trocken-Reforming mit CO2 oder Wasserdampf-Reforming, zu H2-haltigen Synthesegasen umgewandelt werden. Alternative Quellen sind Konvertergas der Eisenproduktion (CO, CO2), besonders in dessen Kombination mit Kokereigas der Eisengewinnung (H2, CH4), ebenso überschüssiges CO2 aus der Zuckerproduktion, CO2-Abgas aus der Zementproduktion, Quellkohlensäure, sowie CO2 und CO aus Verbrennungsgasen (Abgas und Abgaswäscher). Eine CO2-haltige Gasfraktion kann also mit einer anderweitig, insbesondere regenerativ gewonnenen H2-Fraktion angereichert werden, um das geeignete Synthesegasgemisch zu erhalten. Eine bevorzugte Quelle von H2 ist die Elektrolyse, insbesondere in Photovoltaikanlagen oder Kokereigas oder die trockene oder Wasserdampf-Reformation.

In bevorzugter Ausgestaltung ist das CO2-haltige Synthesegas eine Mischung aus zumindest CO2 und H2 und CO, welches in besonderer Variante zusätzlich weitere Gase enthält, welche sich im Wesentlichen bei einer Methanolsynthesereaktion inert verhalten. Dazu zählen besonders N2, Ar und auch CH4.

In einer bevorzugten Ausführung, wird ein stöchiometrisches Verhältnis (SR) eingehalten, welches definiert ist durch:

SR = (C[H2]-C[CO2])/(C[CO]+C[CO2]).

Dabei betrifft der Klammerausdruck "C[ ]" die molare Konzentration der genannten Komponente. Dabei beträgt das stöchiometrische Verhältnis (SR) von 1 bis 3, vorzugsweise von 1,5 bis 2,5, besonders bevorzugt von 1,8 bis 2,3, weiter bevorzugt zwischen 1,8 und 2,3, mehr als 1,8 und weniger als 2,3.

In einer bevorzugten Ausführung ist das CO2-haltige Substrat im wesentlichen frei von O2, H2S, NH3, COS, Aminen, flüchtigen Metallverbindungen, Halogenverbindungen, Schwefelverbindungen oder Verbindungen von Elementen der sechsten Hauptgruppe (Chalkogene). Weiter ist das CO2-haltige Substrat vorzugsweise im Wesentlichen oder vollständig frei von Teer oder Teerbestandteilen. Ein im Wesentlichen nur CO2 und H2 enthaltendes Synthesegas kann in bevorzugter Ausgestaltung dadurch erhalten werden, dass einerseits H2 insbesondere durch Wasserelektrolyse erhalten wird und dass CO2 erhalten wird aus einer oder mehreren CO2-Quellen der Gruppe, bestehend aus:
- hochkonzentriertem CO2-Gasstrom aus der Fermentation von zuckerhaltigen Substraten zu Ethanol-haltigen Produkten oder Flüssigkeiten, aus der Fermentation von zuckerhaltigen Substraten zu Milchsäure-haltigen Substraten, aus der Fermentation von zuckerhaltigen Substraten von 2,3-Butandiol, aus der Fermentation von Methanol-haltigen Substraten zu organischen Verbindungen; aus der Milchverarbeitung; aus der Rückgewinnung von CO2 aus der Atmosphäre; aus der Entgasung von Mineralwasser aus Mineralwasserquellen oder heißen Quellen; aus Biogas; aus der Kalksteinverarbeitung und Zementproduktion; aus der Verbrennung von natürlich vorkommenden Gasen, von synthetischen Gasen, von Biomasse, von Biogas, von Holz, von raffinierten oder unraffinierten Erdölfraktionen, von Crackprodukten, von Erdölfraktionen; und aus der Abfallverbrennung.

In einer alternativen Ausgestaltung wird CO2-, CO- und H2-haltiges Synthesegas erhalten aus einem oder mehreren der nachfolgend genannten Gruppe der Synthesegasquellen, bestehend aus: Synthesegas aus der Dampfreformierung, der autothermalen Reformierung oder der Trockenreformierung von Biogas; aus der Biomassevergasung, der Biomassepyrolyse; aus der Vergasung von Produkten unvollständiger Karbonisierung (Semi-Koks) wie sie bei der Pyrolyse erhalten werden, aus der Vergasung in Gegenwart von H2; aus der Dampfreformierung, autothermalen Reformierung, Trockenreformierung, adsorptionsgesteigerten Reformierung von Biomasse, von Pyrolyseöl oder von Teer aus Biomasse; aus der Dampfreformierung, autothermalen Reformierung oder Trockenreformierung von Alkoholen, biogenen Ölen und Fetten; aus der Dampfreformierung, autothermalen Reformierung oder Trockenreformierung von Erdgas; aus der partiellen Oxidation von Erdgas; aus der Dampfreformierung, autothermalen Reformierung, Trockenreformierung oder adsorptionsgesteigerten Reformierung oder teilweisen Oxidation von Mineralöldestillaten oder Mineralöl-Crackprodukten; aus Koksofengasen; aus Rauchgasen von Hochöfen; aus der Vergasung von Kohle, der Pyrolyse von Kohle, der Vergasung von Semi-Koks, erhältlich aus der Kohlepyrolyse; und aus der Dampfreformierung, autothermalen Reformierung, Trockenreformierung oder adsorptionsgesteigerten Reformierung von Kohleteerdestillaten. Dabei sind bevorzugt aus dem Synthesegas Störsubstanzen wie O2, H2S, NH3, COS, Teer oder Teerprodukte, und flüchtigen Metallverbindungen, Halogenverbindungen, Schwefelverbindungen oder Verbindungen von Elementen der sechsten Hauptgruppe (Chalkogene) im Wesentlichen oder vollständig entfernt.

Im Zusammenhang mit der Einstellung des bevorzugten stöchiometrischen Verhältnisses (SR) kann das zunächst erhaltene Synthesegas eingestellt werden durch Wassergas-Shift-Reaktion, durch umgekehrte Wassergas-Shift-Reaktion, durch Hinzudosieren von H2 aus anderen Quellen (z.B. Wasserelektrolyse), aus Ammoniakabbau oder aus Abbau von flüssigen organischen Wasserstoffträgern.

Unter einem "Methanol-haltigen Gas" wird hier das mittels heterogener Katalyse aus dem vorbezeichneten CO2-haltigen Synthesegas enthaltene gasförmige Produktgemisch verstanden, welches Methanol (CH3OH) als Hauptbestandteil enthält. Weitere Bestandteile in diesem Gasgemisch können nicht umgesetztes Synthesegas sowie Nebenprodukte, das heißt insbesondere CO2, CO und H2 sowie Dimethylether (DME), sein. Nebenprodukte können neben DME auch Ethanol und andere sein.

Unter einem "Methanol-haltigen Substrat", wird hierin das flüssige Gemisch verstanden, das durch Kondensation, das heißt Abkühlen aus dem Methanol-haltigen Gas erhalten wird, wobei gegenüber dem Methanol-haltigen Gas insbesondere die nicht kondensierbare Fraktion, das heißt die nicht verflüssigbaren Gaskomponenten, allem voran CO2, CO und H2, in der Gasphase verblieben und abgetrennt wurden und daher fehlen. Weitere kondensierbare Nebenprodukte der Methanolsynthese können in dem Methanol-haltigen Substrat aber enthalten sein. In einer alternativen Ausgestaltung ist das Methanol-haltige Substrat reines Methanol. Das Substrat wird in dem Kondensator der erfindungsgemäßen Vorrichtung vorzugsweise in wässrige Lösung gebracht und kann so am Kondensator abgezogen und unmittelbar in die nachfolgende Prozessstufe, die biologische Fermentation eingeleitet werden. Die Erfinder fanden überraschend, dass dieses Methanol-haltige Substrat, unmittelbar als Substrat für Methanol-fixierende Mikroorganismen eingesetzt werden kann, um daraus biotechnologisch organische Energiespeicher und/oder Feinchemikalien enthalten.
Dabei sind auch an sich bekannte Methanol-fixierende Mikroorganismen einsetzbar.

Unter "chemischem Energiespeicher" werden vorwiegend organische Moleküle verstanden, die einen hohen Energiegehalt besitzen und später insbesondere in oxidativen Prozessen, das heißt Verbrennung oder Atmung, zur Energierückgewinnung eingesetzt werden können. Dies sind insbesondere höherwertige Alkane, Alkohole oder organischen Säuren, aber auch insbesondere Feinchemikalien wie Terpene, insbesondere Caroten, α-Pinen und Isopren, oder Polyhydroxyalkanoate, beispielsweise zur Herstellung von Biopolymeren.
(1) in einem ersten Schritt wird das CO2-haltige Synthesegas bereitgestellt.
(2) in einem zweiten Schritt wird aus dem Synthesegas ein Methanol-haltiges Gas synthetisiert, und zwar an insbesondere kupferbasierten heterogenen Kontakten. Dabei ist eine isothermale Temperaturführung bevorzugt, wobei die Reaktionswärme durch spezifische Dimensionierung von Reaktionszone und Katalysator kontrolliert wird. Die Reaktionstemperatur der Katalyse beträgt bevorzugt mehr als etwa 250 °C, insbesondere von 250 °C bis 300 °C, bevorzugt aber stets mehr als 250 °C und bis maximal 300 °C. In einer besonderen Ausführungsform beträgt die Reaktionstemperatur zwischen 200 °C und 300 °C, vorzugsweise zwischen 220 °C und 290 °C, besonders bevorzugt zwischen 240 und 290 °C.

Dabei ist erfindungsgemäß vorgesehen, dass diese Reaktion drucklos, das heißt bei etwa Atmosphärendruck, oder alternativ bei Niederdruck oder schwachem Überdruck bis weniger als 10 bar Überdruck, bevorzugt bis weniger als 5 bar Überdruck, besonders bevorzugt bis weniger als 3 bar Überdruck durchgeführt. In einer besonderen Variante beträgt der Reaktionsdruck stets weniger als 150 bar Überdruck, bevorzugt weniger als 100 bar, besonders bevorzugt weniger als 50 bar. Besonders für dezentrale Anlagen beträgt der Reaktionsdruck etwa Atmosphärendruck. Besonders bevorzugt ergibt sich dadurch eine Verbesserung der Gesamtenergiebilanz spezifisch bei kleinformatigen dezentralen Anlagen, welche besonders bei Atmosphärendruck oder leichtem Überdruck, das heißt besonders weniger als 20 bar Überdruck, vorzugsweise weniger als 15 bar Überdruck und besonders bevorzugt weniger als 10 bar Überdruck, betrieben werden können.

Es hat sich nämlich überraschend gezeigt, dass bereits bei diesen Niederdruckbedingungen eine ausreichend hohe Spezifität und Selektivität der heterogenen Katalyse vorherrscht, so dass daraus ein Methanol-haltiges Produktgemisch erhalten werden kann, welches unmittelbar der aeroben Fermentation durch an sich bekannte Methanol-fixierende Mikroorganismen zugeführt werden kann, sofern zuvor lediglich nicht kondensierbare Restgase wie CO und H2, oder CO2, CO2-haltiges und H2, abgetrennt werden. Durch diese Verfahrensführung ist es möglich, eine im Prinzip drucklose oder mit stets nur mit geringem Druckimpulseinrichtung (Niederdruck) betreibbare integrale Anlage zur kombinierten chemokatalytischen und biokatalytischen Synthese bereitzustellen, worin sämtliche Komponenten unmittelbar hintereinander geschaltet und integrierbar sind. Aufgrund der Verfahrensführung bei Atmosphärendruck oder Niederdruck können energieaufwändige Pumpen und Druckaufbringungssysteme in der Anlage vollständig vermieden werden, was die Gesamtenergiebilanz signifikant günstig beeinflussen kann. Die erhöhte Reaktionstemperatur von 250 °C oder mehr, besonders im Bereich zwischen 200 °C und 300 °C, bevorzugt zwischen 220 °C und 290 °C, besonders bevorzugt zwischen 240 °C und 290 °C kann unmittelbar aus der Reaktionswärme der Synthese aufrechterhalten werden.
(3) in einem nachfolgenden weiteren Schritt wird das so erhaltene Methanol-haltige Gas durch Abkühlen kondensiert, wobei nicht kondensierbare Restgase abgetrennt und abgeführt werden. Die Kondensation des Gases erfolgt dabei bevorzugt durch Einleiten in gekühlte wässrige Lösung, besonders Wasser, wodurch schließlich eine wässrige Lösung eines Methanol-haltigen Substrats gewonnen wird.
(4) in einem nachfolgenden weiteren Schritt wird das so kondensierte flüssige Methanol-haltige Substrat unmittelbar in einen Fermentationsreaktor geführt oder dosiert, und es werden in diesem Fermentationsreaktor Methanol-fixierende Mikroorganismen in Zellkulturmedium kultiviert, und zwar unter Bedingungen der aeroben Fermentation, wobei die zugeführten Methanol-haltigen Substrate in organische Energiespeichermoleküle oder in Feinchemikalien umgewandelt werden. Anschließend wird das erhaltene Produktgemisch aus den Zellen und/oder dem Zellkulturmedium abgetrennt und isoliert.

Gegenstand der Erfindung ist auch ein mittels dieses Verfahrens, vorzugsweise unter Verwendung der erfindungsgemäßen Vorrichtung, aus dem CO2-haltigen Synthesegas herstellbares Gemisch organischer Energiespeichermoleküle oder ein Terpen-haltiges Produktgemisch.

Die Erfindung wird durch die Figuren und nachfolgende Beschreibung spezifischer Ausführungsbeispiele näher beschrieben.

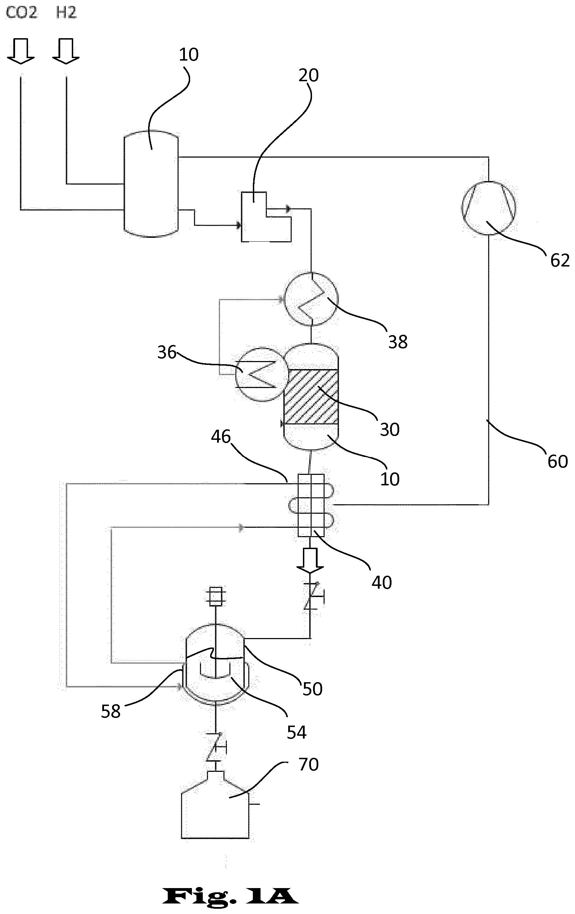

Figur 1A zeigt den prinzipiellen Aufbau der erfindungsgemäßen Vorrichtung in schematischer Darstellung: einem Niederdruckgasreaktor 30 oder Methanolsynthesereaktor zur heterogenen Katalyse ist eine Gasheizeinheit 38 vorgeschaltet, welche bevorzugt über einen Wärmetauscher 36 an dem Niederdruckgasreaktor 30 mit der Reaktionswärme der heterogenen Katalyse gespeist wird, um das Synthesegas vor Eintritt in die Katalyse vorzuheizen. Dem Niederdruckgasreaktor 30 ist eine Gasdosiereinheit 20 vorgeschaltet, die aus einer Gasspeichereinheit 10 gespeist wird. Dem Niederdruckgasreaktor 30 nachgeschaltet ist ein Kondensator 40, der als Kühlfalle dient, um das aus dem Niederdruckgasreaktor 30 austretende Methanol-haltige Gasprodukt abzukühlen und daraus kondensierbare Komponenten, allen voran Methanol, zu kondensieren, das heißt zu verflüssigen. Das verflüssigte Methanol-haltige Substrat wird über ein Dosierventil dem unmittelbar nachgeschalteten Fermentationsreaktor 50 zugeführt, wohingegen nicht kondensierbare Restgase in den Gasspeicher 10 über eine separate Rückleitung 60 zurückgepumpt werden. Dies geschieht vorzugswiese durch eine aktive Niederdruckpumpe 62 in der Rückleitung 60. Der Kondensator 40 ist aktiv durch einen Kühlmantel (Kühlschlange) mit Wärmetauscher 46 gekühlt. Die dort abgezogene Wärme wird einer an dem Fermentationsreaktor 50 angeordnete Medienheizeinheit 58 zugeführt, die dort zum Temperieren des Zellkulturmediums dient. In dem Zellkulturmedium des Fermentationsreaktors 50 befinden sich Methanol-fixierende Mikroorganismen 54. Bei der aeroben Fermentation wandeln diese Methanol-fixierenden Mikroorganismen, dass über dem Kondensator unmittelbar zugeführte Methanol-haltige Substrat in organische Energiespeicher oder in Feinchemikalien wie Terpen-haltige Produktgemische um, die in einer Ernte- und Trenneinheit 70 aus der Fermentation isoliert werden können

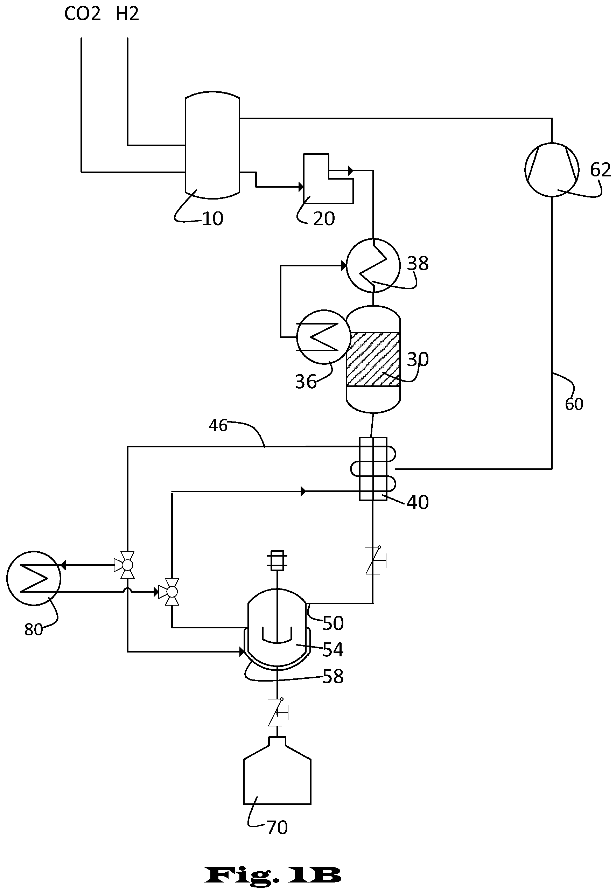

Figur 1B zeigt eine besondere Ausführung der Vorrichtung nach Figur 1A, wobei in dem Wärmetauschkreis 46 zum Wärmeaustausch an dem Kondensator 40 ein weiterer Wärmetauscherkreis 80 im Nebenschluss zuschaltbar ist. Der Wärmetauscher 80 dient, insbesondere bei kontinuierlichem Betrieb des Kondensators 40 zur Abfuhr zusätzlicher Wärme aus dem System, also vorallem von derjenigen Reaktionswärme, die nicht zur Temperierung des Mediums 54 in dem Fermentationsreaktor 50 benötigt wird.

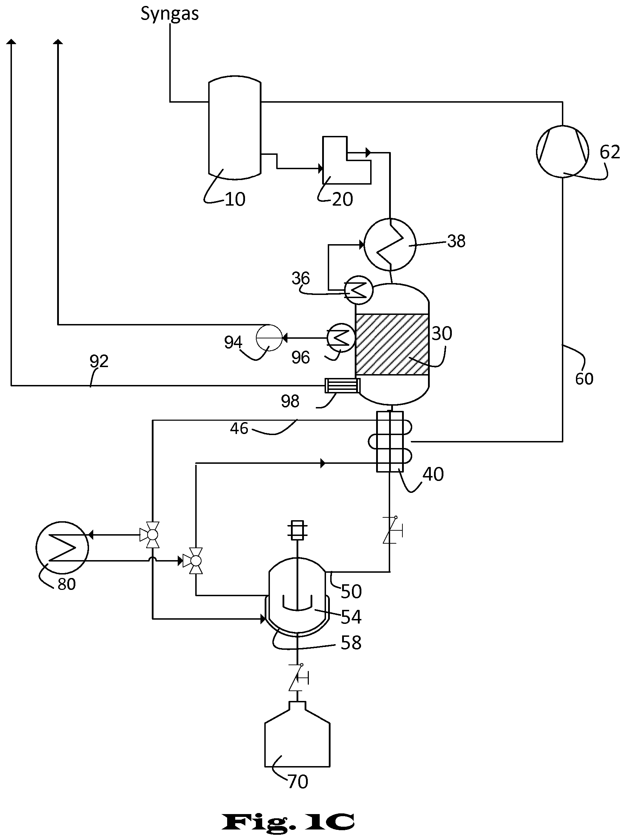

Figur 1C zeigt eine andere besondere Ausführung der Vorrichtung nach Figur 1A beziehungsweise 1B, wobei neben dem Wärmetauscher 36 zur Rückführung von Reaktionswärme in das System über Wärmetauscher 38 an dem Reaktor 30 ein zusätzlicher Wärmetauscher 96 vorgesehen ist, der mit einem Dampferzeuger 94 in Verbindung steht, wobei die aus dem Reaktor 30 dadurch abgeführte Reaktionswärme zur Dampferzeugung herangezogen werden kann. Der Dampf wird aus dem Dampferzeuger abgezogen und kann zur Vorheizung des Synthesegases (Syngas) verwendet werden. Der mittels System 96, 94 erzeugte Dampf kann auch herangezogen werden um einen Niederdruck- bis Mitteldruck-Dampf zu erzeugen, welcher zur Generierung des Synthesegases herangezogen werden kann. Alternativ oder zusätzlich ist ein Hochdruck-Dampfreaktor 98 an dem Reaktor 30 vorgesehen, welcher durch die Reaktionswärme aus dem Reaktor 30 gespeist wird. Hochdruck-Dampf kann dann über Leitung 92 abgezogen werden und zur Erzeugung des Synthesegases verwendet werden.

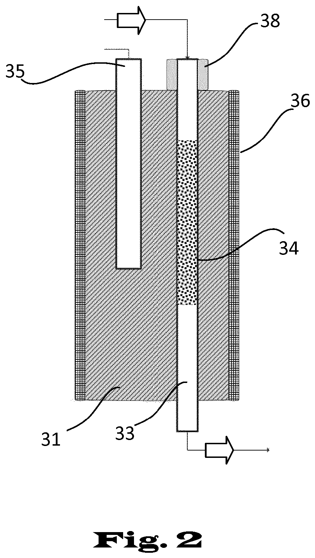

Figur 2 zeigt eine schematische Darstellung einer Ausführung des erfindungsgemäßen Niederdruckgasreaktors 30: In einem wärmeleitenden Reaktorblock 31 aus Aluminium ist ein Reaktionsrohr 33 aus Quarzglas eingesetzt, worin sich der partikuläre Katalysator 34 befindet. In das Reaktionsrohr 33 tritt (im Bild oben) das in dem Gasheizer 38 vorgeheizte Synthesegas ein. Am Ausgang des Reaktors (im Bild unten) kann das synthetisierte Methanol-haltige Gas abgezogen werden. In dem dargestellten Reaktorblock 31 ist zusätzlich eine Heizpatrone 35 vorgesehen, welche initial den Reaktor auf Reaktionstemperatur bringen kann, um die Reaktion zu starten. Der Reaktorblock ist von einem Isoliermantel umgeben, der als Wärmetauscher 36 dient, um Reaktionswärme aus dem Reaktor abzuführen.

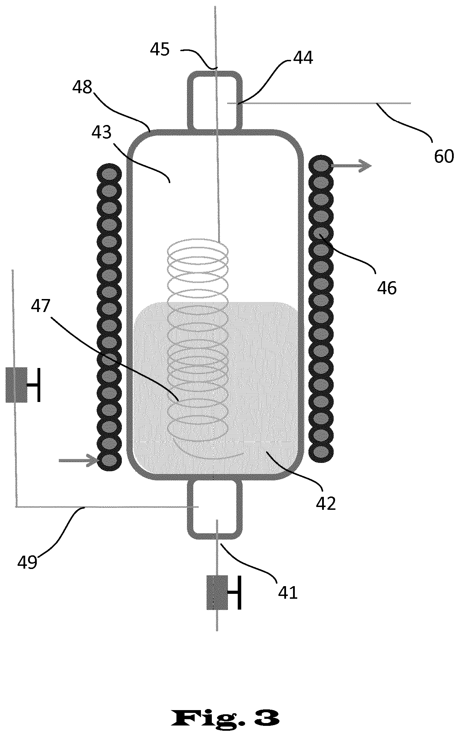

Figur 3 zeigt eine schematische Darstellung einer Ausführung des erfindungsgemäßen Kondensators 40. In eine im Wesentlichen zylindrische Kammer 38 ist ein schraubenartig gebogenes, am Ende offenes Gasrohr 47 eingehängt, über welches Methanol-haltiges Gas zugeführt werden kann. Dazu ist das Gasrohr 47 mit einem Anschluss 45 verbunden. In dem oberen, im Betrieb des Kondensators 40 gasgefüllten, Gas-Abschnitt 43 ist ein oberer Anschluss 44 vorgesehen, der spezifisch ausgebildet ist, nicht verflüssigbare Restgase abzuleiten, und zwar über die Rückleitung 60. In dem unteren, im Betrieb flüssigkeitsgefüllten, Flüssigkeits-Abschnitt 42 taucht das Gasrohr 47 in die Flüssigkeit ein. Dort kondensiert das Methanol-haltige Gas zu einem Methanol-haltigen Substrat, welches über den unteren Anschluss 41 zum Abführen des verflüssigten Substrates abgezogen werden kann. Der Mantel der Kammer 48 ist über einen als Kühlschlange ausgebildeten Wärmetauscher 46 aktiv gekühlt. Über eine Leitung 49 kann die Flüsigkeit vorgelegt, aber auch Proben aus dem laufenden Verfahren gezogen werden.

### Beispiel: Kombinierte chemische und biotechnologische Synthese von Terpenen aus einem Gasgemisch aus CO2 und H2

In einem Gasspeichertank werden die Gase CO2 und H2 aus verschiedenen Quellen in einem Molverhältnis von 1:3 vorgelegt. Gas wird über einen Massendurchfluss-Regler (Bronkhorst MFC) in einen beheizten Rohrreaktor dosiert. Die Gasflussrate des CO2 beträgt dabei von 2,5 bis 50 ml(n)/min, der Gasfluss von H2 beträgt dabei von 10 bis 200 ml(n)/min. Der Druck der Gase aus der Vorlage vor dem Massenfluss-Regler beträgt etwa 3 bar; nach Durchfluss des Massenfluss-Reglers treten die Gase bei etwa Atmosphärendruck in den Rohrreaktor ein. Ein Überdruck von 20-30 mbar reicht aus, um den Gasfluss durch den Reaktor aufrechtzuerhalten. Die an sich volumenfördernde Synthesereaktion führt bei Betrieb mit Atmosphärendruck zu vernachlässigbaren Effekten.

Der Niederdruckreaktor besteht aus einem zylindrischen mit einer elektrischen Heizpatrone vorgeheizten Aluminiumblock, worin ein mit partikulärem Katalysatormaterial gefülltes Quarzrohr, Durchmesser 10 mm, eingebettet ist. Die Reaktionszone in dem Quarzrohr hat eine Länge von etwa 15 cm. Die Reaktorkomponente sind so dimensioniert, dass eine isothermale Reaktionsführung ermöglicht wird. Im Labormaßstab wird der Stoffdurchsatz durch Parallelschalten mehrerer Reaktorrohre in einem Block erhöht.

Die Temperatur innerhalb des Rohrreaktors beträgt während der Reaktion etwa 250 °C, die heterogenkatalytische Synthesereaktion läuft isothermal ab. Die im Reaktor entstehende Reaktionswärme wird abgeführt und beheizt das Synthesegas vor Eintritt in den Reaktor auf etwa 120° C. In einem alternativen Ansatz wird statt der elektrischen Vorheizung des Rohrreaktors eine Heizung mit Hochtemperaturwasserdampf oder eine Ölheizung installiert. In einem alternativen Ansatz wird das Katalysatormaterial direkt über Mikrowellenstrahlung erhitzt.

Der heterogene Katalysator in dem Rohrreaktor ist ein partikulärer Kupfer-Mischmetallkatalysator auf Zinkoxid (ZnO) und Alumina (Al2O3) mit einem Anteil von etwa 30 bis 70 Gew.-% Cu, etwa 20 bis 50 Gew.-% Zn und etwa 10 bis 20 Gew.-% Al, wobei der Kupferkontakt möglichst nanopartikulär mit einer Metallpartikelgröße von 1 bis 100 nm vorliegt. Der Katalysator wird in an sich bekannter Weise aus den Salzen Kupfernitrat (Trihydrat), Zinknitrat (Hexahydrat) und Aluminiumnitrat (Nonahydrat) gefällt. Die mittlere Größe der Katalysatorkörner beträgt 200 bis 320 µm

Das aus dem Rohrreaktor austretende Reaktionsprodukt, ein Methanol-haltiges Gasgemisch mit Restgasen, wird in einer unmittelbar nachgeschalteten Kühlfalle verflüssigt. Die Kühlfalle besteht aus einem zylindrischen Kolben mit Mantelkühlung. In dem Kolben taucht ein schraubenförmig gebogenes Gasrohr in etwa 15 ml vorgelegtes Wasser. Das Methanol-Gas wird dort durch Abkühlen verflüssigt und Methanol und gegebenenfalls Nebenprodukte lösen sich darin. Die Methanol-Konzentration in der so erhaltenen wässrigen Lösung beträgt etwa von 1,5 bis 15 mmol/L. Sich nicht lösende und nicht kondensierbare Restgasbestandteile werden oben am Reaktorkolben abgezogen und über eine Rückleitung in die Gasvorlage zurückgepumpt. Die wässrige Lösung des Methanol-haltigen Substrats wird am Kolben unten abgezogen und in den unmittelbar damit verbundenen Fermentationsreaktor dosiert.

Diese Kühlfalle wird im Batch-Verfahren betrieben: In einer ersten Phase in dem Kolben Flüssigkeit vorgelegt wird, in einer zweiten Phase in die gekühlte Flüssigkeit das Methanol-haltige Gasgemisch eingeleitet und dort kondensiert wird. In einer dritten Phase wird über extern zugeführtes inertes Spülgas (N2) anstelle des Methanol-haltigen Gasgemisches aus dem Reaktor durch das in das Flüssigkeit getauchte Rohr eingepumpt, um nicht kondensierendes Restgas, insbesondere bestehend aus Katalyse-Nebenprodukten wie Kohlenstoffmonoxid (CO) und Dimethylether (DME), sowie aus nicht reagiertem Synthesegaskomponenten (CO2, H2) aus der Kühlfalle auswärts getrieben und insbesondere über die Rückleitung in die Vorlage zurückgeführt.

In den Fermentationsreaktor ist ein Zellkulturmedium vorgelegt, das mit Mikororganismen der Gattung Methylobacterium extorquens (AM1) inokuliert ist. Die Bakterien sind spezifisch ausgewählt, um aus dem Methanol-haltigen Substrat als Kohlenstoffquelle Terpene, insbesondere Carotene zu bilden. Diese werden in den Mikroorganismen akkumuliert. Die Kultivierung findet in fed-batch-Modus bei einer Temperatur von etwa 20 bis 28 °C unter konstanter Durchmischung und Begasung mit gereinigter Pressluft statt. Die Kultivierungsdauer beträgt 1 bis 3 Tage.

Im Anschluss an die Kultivierung werden die akkumulierten Carotene aus den Mikroorganismen extrahiert. Dazu werden die Zellen aus dem Kulturmedium geerntet und in an sich bekannter Weise mit DMSO vermischt, aufgeschlossen und zentrifugiert. Im Pellet wird Caroten erhalten, welches anschließend getrocknet werden kann.

Die Carotenmenge wird spektrophotometrisch (510 nm) über Korrelation mit Verleichssubstanz (getrocknetes Caroten) bestimmt. Es werden etwa 10 bis 30 mg Caroten pro Gramm Biomasse erhalten. Die Gesamtausbeute der Fermentation beträgt 6 mg Caroten pro 1g Methanol-Substrat.

## Patentansprüche

1. Vorrichtung zur integralen Synthese von organischen Energiespeichern aus Kohlenstoffdioxid-haltigem Synthesegas, enthaltend in Stoffflussrichtung unmittelbar hintereinander geschaltete Elemente:
- Niederdruckgasreaktor (30) mit kupferbasiertem heterogenem Katalysator (34) zur katalytischen Synthese eines Methanol-haltigen Gases aus dem Synthesegas,
- dem Niederdruckgasreaktor (30) unmittelbar nachgeschalteter Kondensator (40) zur Verflüssigung dieses Methanol-haltigen Gases zu einem flüssigen wässrigen Methanol-haltigen Substrat und Ableitung nicht verflüssigbarer Restgase,
- dem Kondensator (40) unmittelbar nachgeschalteter Fermentationsreaktor (50), enthaltend flüssiges Zellkulturmedium und Methanol-fixierende Mikroorganismen (54) zur aeroben Fermentation Methanol-haltiger Substrate zu organischen Energiespeichern, zur Aufnahme dieses kondensierten flüssigen wässrigen Methanol-haltigen Substrats.

2. Vorrichtung nach Anspruch 1, weiter enthaltend eine dem Niederdruckgasreaktor (30) unmittelbar vorgeschaltete Gasdosiereinheit (20).

3. Vorrichtung nach Anspruch 2, weiter enthaltend eine der Gasdosiereinheit (20) unmittelbar vorgeschaltete Gasspeichereinheit (10).

4. Vorrichtung nach Anspruch 3, weiter enthaltend eine Rückleitung (60) zwischen Kondensator (40) und Gasspeichereinheit (10) zum Rückführen der nicht verflüssigbaren Restgase aus dem Kondensator (40) in die Gasspeichereinheit (10).

5. Vorrichtung nach einem der vorstehenden Ansprüche, weiter enthaltend eine dem Fermentationsreaktor (50) nachgeschaltete Stofftrenneinheit (70) zum Abtrennen der synthetisierten organischen Energiespeicher aus den Mikrorganismen und/oder dem Zellkulturmedium.

6. Vorrichtung nach einem der vorstehenden Ansprüche, weiter enthaltend eine dem Niederdruckgasreaktor (30) unmittelbar vorgeschaltete oder darin integrierte Gasheizeinheit (38) zum Vorheizen des Synthesegases vor oder bei Eintritt in die Katalyse, wobei die Gasheizeinheit (38) von der in dem Niederdruckgasreaktor (30) entstehenden Reaktionswärme über einen diesem zugeordneten Wärmetauscher (36) gespeist wird.

7. Vorrichtung nach einem der vorstehenden Ansprüche, weiter enthaltend eine dem Fermentationsreaktor (50) zugeordnete oder darin integrierte Medienheizeinheit (58) zum Temperieren des Zellkulturmediums in dem Fermentationsreaktor (50), wobei die Medienheizeinheit (58) von der aus dem Kondensator (40) abzuführenden Wärme über 10 einen diesem zugeordneten Wärmetauscher (46) gespeist wird.

8. Vorrichtung nach einem der vorstehenden Ansprüche, wobei der Kondensator (40) weiter enthält:
- eine Kammer (48) mit oberem Gas-Abschnitt (43) mit einem oberen Anschluss (44) zum Ableiten nicht verflüssigbarer Restgase und unterem Flüssigkeits-Abschnitt (42) mit einem unteren Anschluss (41) zum Abführen des verflüssigten Methanol-haltigen Substrates,
- ein mit einem Anschluss (45) verbundenes und in den unteren Flüssigkeits-Abschnitt (42) eintauchendes Gasrohr (47) zum Einleiten des gasförmigen Methanol-haltigen Substrates in den Flüssigkeits-Abschnitt (42) der Kammer (48).

9. Verfahren zur integralen Synthese von organischen Energiespeichermolekülen aus Kohlenstoffdioxid-haltigem Synthesegas, enthaltend die Schritte:
- Bereitstellen von Synthesegas, enthaltend CO2 und H2;
- Synthese von Methanol-haltigem Gas aus dem Synthesegas an kupferbasiertem heterogenem Katalysator, bei einer Temperatur von 250 bis 300 °C und bei Atmosphärendruck oder Niederdruck von weniger als 5 bar Überdruck;
- Kondensieren des unmittelbar erhaltenen Methanol-haltigen Gases zu einem flüssigen wässrigen Methanol-haltigen Substrat und Ableiten nicht kondensierbarer Restgase;
- unmittelbares Zuführen des flüssigen wässrigen Methanol-haltigen Substrates in einen Fermentationsreaktor;
- Kultivieren von Methanol-fixierenden Mikroorganismen in dem Fermentationsreaktor in Zellkulturmedium und dem flüssigen wässrigen Methanol-haltigen Substrat unter Bedingungen der aeroben Fermentation, geeignet zur Umwandlung zugeführten Methanol-haltigen Substrats in organische Energiespeichermoleküle; und
- Abtrennen der organischen Energiespeichermoleküle aus den Mikroorganismen und/oder dem Zellkulturmedium.

## Claims

1. A device for the integral synthesis of organic stored-energy sources from carbon dioxide-containing synthesis gas, containing elements connected directly in series in the direction of material flow:
- a low-pressure gas reactor (30) with a copper-based heterogeneous catalyst (34) for the catalytic synthesis of a methanol-containing gas from the synthesis gas,
- a condenser (40) connected directly downstream of the low-pressure gas reactor (30) for liquefying this methanol-containing gas into a liquid aqueous methanol-containing substrate and for discharging non-liquefiable residual gases,
- a fermentation reactor (50) connected directly downstream of the condenser (40), containing liquid cell culture medium and methanol-fixing microorganisms (54) for the aerobic fermentation of methanol-containing substrates to organic stored-energy sources, for receiving this condensed liquid aqueous methanol-containing substrate.

2. The device of claim 1, further containing a gas dosing unit (20) connected directly upstream of the low-pressure gas reactor (30).

3. The device of claim 2, further containing a gas storage unit (10) connected directly upstream of the gas dosing unit (20).

4. The device of claim 3, further containing a return line (60) between the condenser (40) and the gas storage unit (10) for returning the non-liquefiable residual gases from the condenser (40) to the gas storage unit (10).

5. The device of one of the preceding claims, further containing a substance separating unit (70) connected downstream of the fermentation reactor (50) for separating the synthesised organic stored-energy sources from the microorganisms and/or the cell culture medium.

6. The device of one of the preceding claims, further containing a gas heating unit (38) connected directly upstream of or integrated into the low-pressure gas reactor (30) for preheating the synthesis gas before or upon entry into the catalysis, wherein the gas heating unit (38) is supplied with the reaction heat generated in the low-pressure gas reactor (30) via a heat exchanger (36) assigned thereto.

7. The device of one of the preceding claims, further containing a media heating unit (58) assigned to or integrated into the fermentation reactor (50) for controlling the temperature of the cell culture medium in the fermentation reactor (50), wherein the media heating unit (58) is supplied with the heat to be removed from the condenser (40) via a heat exchanger (46) assigned thereto.

8. The device of one of the preceding claims, wherein the condenser (40) further contains:
- a chamber (48) having an upper gas section (43) having an upper port (44) for discharging non-liquefiable residual gases and a lower liquid section (42) having a lower port (41) for discharging the liquefied methanol-containing substrate,
- a gas pipe (47) connected to a port (45) and immersed in the lower liquid section (42) for introducing the gaseous methanol-containing substrate into the liquid section (42) of the chamber (48).

9. A method for the integral synthesis of organic stored-energy molecules from carbon dioxide-containing synthesis gas, containing the steps:
- providing synthesis gas containing CO2 and H2;
- synthesis of methanol-containing gas from the synthesis gas on a copper-based heterogeneous catalyst at a temperature of 250 to 300 °C and at atmospheric pressure or low pressure of less than 5 bar overpressure;
- condensing the directly obtained methanol-containing gas to a liquid aqueous methanol-containing substrate and discharging non-condensable residual gases;
- directly feeding the liquid aqueous methanol-containing substrate into a fermentation reactor;
- Cultivating methanol-fixing microorganisms in the fermentation reactor in cell culture medium and the liquid aqueous methanol-containing substrate under conditions of aerobic fermentation suitable for converting the fed methanol-containing substrate into organic stored-energy molecules; and
- separating the organic stored-energy molecules from the microorganisms and/or the cell culture medium.

## Revendications

1. Un dispositif pour la synthèse intégrale de réservoirs d'énergie organique à partir d'un gaz de synthèse contenant du dioxyde de carbone, contenant des éléments connectés directement en série dans le sens du flux de matière :
- un réacteur à gaz à basse pression (30) avec un catalyseur hétérogène à base de cuivre (34) pour la synthèse catalytique d'un gaz contenant du méthanol à partir du gaz de synthèse,
- un condenseur (40) connecté directement en aval du réacteur à gaz à basse pression (30) pour liquéfier ce gaz contenant du méthanol en un substrat liquide aqueux contenant du méthanol et pour rejeter les gaz résiduels non liquéfiables,
- un réacteur de fermentation (50) connecté directement en aval du condenseur (40), contenant un milieu de culture cellulaire liquide et des micro-organismes fixateurs de méthanol (54) pour la fermentation aérobie de substrats contenant du méthanol en des réservoirs d'énergie organique, pour recevoir ce substrat liquide aqueux condensé contenant du méthanol.

2. Le dispositif selon la revendication 1, contenant en outre une unité de dosage de gaz (20) connectée directement en amont du réacteur à gaz à basse pression (30).

3. Le dispositif selon la revendication 2, contenant en outre une unité de stockage de gaz (10) connectée directement en amont de l'unité de dosage de gaz (20).

4. Le dispositif selon la revendication 3, contenant en outre une conduite de retour (60) entre le condenseur (40) et l'unité de stockage de gaz (10) pour renvoyer les gaz résiduels non liquéfiables du condenseur (40) vers l'unité de stockage de gaz (10).

5. Le dispositif selon l'une des revendications précédentes, contenant en outre une unité de séparation de substances (70) connectée en aval du réacteur de fermentation (50) pour séparer les réservoirs d'énergie organique synthétisées des micro-organismes et/ou du milieu de culture cellulaire.

6. Le dispositif selon l'une des revendications précédentes, contenant en outre une unité de chauffage de gaz (38) connectée directement en amont du réacteur à gaz à basse pression (30) ou intégrée dans celui-ci pour préchauffer le gaz de synthèse avant ou lors de son entrée dans la catalyse, dans lequel l'unité de chauffage de gaz (38) est alimentée en chaleur de réaction générée dans le réacteur à gaz à basse pression (30) par l'intermédiaire d'un échangeur de chaleur (36) qui lui est associé.

7. Le dispositif selon l'une des revendications précédentes, contenant en outre une unité de chauffage de milieu (58) associée au réacteur de fermentation (50) ou intégrée dans celui-ci pour contrôler la température du milieu de culture cellulaire dans le réacteur de fermentation (50), dans lequel l'unité de chauffage de milieu (58) est alimentée en chaleur à enlever du condenseur (40) par l'intermédiaire d'un échangeur de chaleur (46) qui lui est associé.

8. Le dispositif selon l'une des revendications précédentes, dans lequel le condenseur (40) contient en outre :
- une chambre (48) ayant une section supérieure de gaz (43) avec un orifice supérieur (44) pour rejeter les gaz résiduels non liquéfiables et une section inférieure de liquide (42) avec un orifice inférieur (41) pour rejeter le substrat liquéfié contenant du méthanol,
- un tuyau de gaz (47) connecté à un orifice (45) et immergé dans la section inférieure de liquide (42) pour introduire le substrat gazeux contenant du méthanol dans la section de liquide (42) de la chambre (48).

9. Un procédé pour la synthèse intégrale de molécules organiques de réservoirs d'énergie à partir d'un gaz de synthèse contenant du dioxyde de carbone, comprenant les étapes :
- fournir un gaz de synthèse contenant du CO2 et du H2 ;
- Synthèse d'un gaz contenant du méthanol à partir du gaz de synthèse sur un catalyseur hétérogène à base de cuivre à une température de 250 à 300 °C et à pression atmosphérique ou à basse pression d'au moins de 5 bars de surpression ;
- condenser le gaz contenant du méthanol directement obtenu en un substrat liquide aqueux contenant du méthanol et rejeter les gaz résiduels non condensables ;
- alimenter directement le substrat liquide aqueux contenant du méthanol dans un réacteur de fermentation ;
- cultiver des micro-organismes fixateurs de méthanol dans le réacteur de fermentation dans un milieu de culture cellulaire et le substrat liquide aqueux contenant du méthanol dans des conditions de fermentation aérobie adaptées à la conversion du substrat contenant du méthanol alimenté en molécules organiques de réservoirs d'énergie ; et
- séparer les molécules organiques de réservoirs d'énergie des micro-organismes et/ou du milieu de culture cellulaire.
